# EUROPEAN PATENT SPECIFICATION

(11) **EP 1 330 164 B1**
(45) Date of publication and mention of the grant of the patent: **07.12.2005**
(21) Application number: 01930295.9
(22) Date of filing: 11.05.2001
(51) Int. Cl.: A01N 59/16, A01N 33/02, A01N 47/44, A01N 55/02, A61L 9/01

(54) **ANTIBACTERIAL AGENTS AND ANTIBACTERIAL AND DEODORIZING SOLUTION COMPRISING THE SAME**
ANTIBAKTERIELLE MITTEL SOWIE DIESE ENTHALTENDE ANTIBAKTERIELLE UND DEODORIERENDE LÖSUNG
AGENTS ANTIBACTERIENS ET SOLUTION ANTIBACTERIENNE ET DESODORISANTE LES CONTENANT

(30) Priority: 09.10.2000 KR 2000059345
(43) Date of publication of application: 30.07.2003
(73) Proprietor: KOREA RESEARCH INSTITUTE OF CHEMICAL TECHNOLOGY, Daejeon 305-343 (KR)
(72) Inventor: KIM, Sung Soo, Daejeon 305-390 (KR); PARK, In-Hwan, Yusung-ku, Daejeon 305-340 (KR); SHIN, Byung Chul, Yusung-ku, Daejeon 305-333 (KR); LEE, Soo Hong, Daeduok-ku,306-200 Daejeon (KR)
(74) Representative: Behnisch, Werner
(86) International application number: PCT/KR2001/000766
(87) International publication number: WO 2002/030204

(56) References cited:
- WO-A1-00/15036
- PATENT ABSTRACTS OF JAPAN & JP 09 087 116 A (TOYO INK CO LTD MEISI MILK PROD CO LTD.) 31 March 1997
- PATENT ABSTRACTS OF JAPAN & JP 2000 256365 A (MEISI MILK PROD CO LTD) 19 February 2000
- PATENT ABSTRACTS OF JAPAN & JP 10 273 875 A (KOHJIN CO LTD) 13 October 1998

## Description

### BACKGROUND OF THE INVENTION

### Field of the Invention

The present invention relates to novel antibacterial agents wherin the lone pair electrons of nitrogen atoms of amine compounds with high boiling point or water-soluble polymer with basic nitrogen at the backbone or side chain are coordinated with silver ion, and antibacterial and deodorizing solution comprising them.

Bacteria and molds produce low-molecular compounds emitting offensive odor by decomposing organic compounds like food with the secretion of enzymes. Also, bacteria and molds are sources of offensive odor in clothes, shoes, basement, pets and ditches. Accordingly, there have been continuous attempts to develop antibacterial and deodorizing agents which are effective for deodorization, agricultural deodorizing agent, skin-related antibacterial agent, green algae prevention and cockroach extermination by exterminating microbes like bacteria.
General organic antibacterial agents, among currently used antibacterial agents, exterminate microbes by infiltrating into the microbial body and transforming its DNA. So, there is a possibility to generate resistant microbes. On the other hand, since silver (Ag) compounds exterminate microbes by disturbing the electron-transfer system of the cell membrane outside the microbial body, there is no concern of resistant microbes. Also, there is another advantage that many silver (Ag) compounds usable for antibacterial agents have much lower toxicity than general organic antibacterial agents.
Most silver (Ag) compounds have good antibacterial effect and lower toxicity compared with organic antibacterial agents, and does not induce the generation of resistant microbes. However, although insoluble silver (Ag) compounds like silver chloride (AgCl) and silver iodide (AgI) form uniform colloids and exist in ionic phase in water, precipitates are formed with time due to the inter-colloidal bond. So, they cannot be used in spray or solution form, and therefore the antibacterial effect becomes insufficient.
Soluble silver (Ag) compounds form insoluble salts like silver chloride or silver sulfide by binding with chloride or sulfate ion generally present in water. These silver compounds transform to silver metal through reduction and oxidation by light, and then blackens to silver oxide. If soluble silver (Ag) compounds are dissolved in pure water, the discoloration is delayed. But, color changes after long-time exposure to light. Accordingly, these soluble silver (Ag) compounds are not suitable for use as everyday antibacterial agents.

For the report of everyday antibacterial agent using silver (Ag) compounds up to now, there is an example of forming silver dichloride (AgCl₂) by adding silver salt in chloride salts such as ammonium chloride, alkali metal chloride or alkaline earth metal chloride solution [International Patent Publication WO99/09833]. In this method, silver monochloride which discolors in light is not formed when the concentration of chloride salts is at least 60 times larger than that of silver salt. Accordingly, it is not suitable for spray-type antibacterial agent since it can induce discomfort or skin irritation due to the too high salt concentration.
While silver compounds cannot be used for oral antibiotics because they are not absorbed well into the body, they can be used for antibacterial agents not required to be absorbed into the body, e.g. for the treatment of skin diseases and burns caused by bacteria and molds, since they have a wide antibacterial spectrum. However, general soluble silver compounds have problems of discoloration through the binding with skin protein, and insoluble silver compounds are not suitable for use as antibacterial agents due to their low antibacterial effect. In other words, because silver compounds are easily discolored by light, they may cause discoloration or stain when used for everyday antibacterial agents. Their colors also change when they are dissolved in water to be used for spray, skin-related antibacterial agent, agricultural fungicide, etc. The cause of discoloration is that silver ion is reduced to silver metal by light, and then this silver metal is oxidized to silver oxide by the ambient oxygen.

WO 00/15036 discloses a topical antimicrobial composition containing an antimicrobial complex that provides sustained antimicrobial disinfection action upon contact with organisms for prolonged periods without the necessity for re-application. The composition comprises (i) an antimicrobial complex comprising an organic polycationic polymeric antimicrobial material and an antimicrobial metallic material, wherein the metallic material is non-leachably bound to or associated with said organic polymeric antimicrobial material and (ii) a carrier wherein an antimicrobial complex (i) is dispersed within said carrier (ii). Especially preferred organic materials are polyhexamethylenebiguanide, polyhexamethylenebiguanide hydrochloride, or derivatives thereof. A topical microbial composition provides both initial and residual contact-killing disinfection activity and does not release its antimicrobial components into contacting liquids at levels that result in solution disinfection.

In JP 09-087116-A, an antibacterial and antifungal composition is disclosed, wherein the composition contains both A: a slightly water-soluble compound comprising a complex containing a coordinated silver ion and B: a water-soluble amine. The compound A is preferably a complex formed by coordinating a silver ion in a thio group containing compound. C: a water-dispersible resin may be added to the system.

JP 00-256365 discloses a water-soluble and light stable silver complex showing antibacterial and antifungal activity. This complex is prepared by coordinating a) 2-pyrolydon-5 caboxylic acid or b) histidine to c) silver ion at a suitable pH in an aqueous solution.

Also, the silver compounds can discolor skins when used as skin-related antibacterial agents.

### SUMMARY OF THE INVENTION

Generally, the reduction-oxidation reactions of soluble silver compounds in pure water without anions like distilled water proceed slowly, and therefore the discoloration time is rather long. However, general industrial water and tap water contain anions like chloride ion or sulfate ion, and they react with silver compounds to produce insoluble silver chloride or silver sulfide. These insoluble silver compounds reduce to silver metal due to the light energy of UV etc., and then oxidize by ambient oxygen to produce silver oxide and discolor the solution. So, they are not suitable for use as antibacterial deodorizing agent, agricultural antibacterial agent, green algae inhibitor, etc. Also, the soluble silver compounds may discolor the skin by binding with the skin protein and producing silver oxide when they are used for skin-related treatments.

The inventors made efforts to resolve the problems of discoloration due to light and skin discoloration while utilizing the unique antibacterial effect, low toxicity and nonresistance of silver compounds or silver ions. In doing so, aiming at the properties of silver (Ag) compounds and silver ions, we prepared silver complex with novel structure. This novel compounds has superior antibacterial effect, low toxicity, good solubility to water and stable silver ion. So, since it neither discolors by light nor discolors the skin, it can be prepared as antibacterial deodorizing solution suitable for antibacterial agents for clothes or skin treatment.
Accordingly, an object of the present invention is to provide novel antibacterial agents and antibacterial and deodorizing solution comprising them, which have superior antibacterial effect and low toxicity, and hardly discolor by light.

### Brief Description of Figures

FIG. 1 is a photograph which shows the discoloration of the silver nitrate solution and silver-polyethyleneimine complex solution after exposing them for 2hr in UV.
FIG. 2 is a photograph which shows the behavior of cockroaches in the part where 2%-diluted solution of silver-polyethyleneimine complex was sprayed and in the non-treated part.

### Detailed Description of the Invention

The present invention is characterized by a silver complex antibacterial agent wherin the lone pair electrons of nitrogen atoms of amine compounds with high boiling point or water-soluble polymer with basic nitrogen at the backbone or side chain are coordinated with silver ion.
Another characteristic of the present invention is an antibacterial and deodorizing solution comprising the said silver complex antibacterial agent.

Hereunder is given the more detailed description of the present invention.
The present invention relates to: a silver complex antibacterial agent which stabilizes silver ion to prevent its reaction with anions like chloride ion or sulfate ion present in water and prevents the reduction-oxidation reaction, and therefore does not discolor in general water other than distilled water; and antibacterial and deodorizing solution comprising it.
The following Formula (1) represents a silver complex wherein the polyethyleneimine polymer is coordinated with silver ion, and it is a typical example of many antibacterial agents included in the present invention.

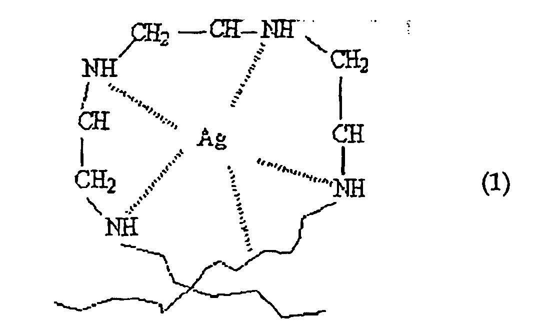

Generally, if ammonia (NH₃) is added in aqueous solution containing soluble silver compound, the discoloration is prevented. This is because the silver ion binds with the lone pair electrons of basic nitrogen to form a complex, that is suitable in ionic form itself and dissolves silver chloride which is a photo-discoloring hardly-soluble silver compound. However, ammonia (NH₃) is not suitable for use as spray-type antibacterial agent, because it emits very offensive odor. In comparison, the silver complex according to the present invention provides the silver ion stabilization effect as in the addition of ammonia (NH₃) and does not emit the offensive odor. So, it is useful for spray-type antibacterial and deodorizing agent.

In the present invention, for the basic nitrogen containing inaterial, amine compounds with high boiling point or water-soluble polymers with plenty of basic nitrogen atoms having lone pair electrons at the polymer backbone or side chain are used.
The amine compounds with high boiling point are adequate for the purpose of the present invention since they emit hardly any offensive odor. For the amine compounds with high boiling point of the present invention, amine compounds with high boiling point selected from monoethanolamine, diethanolamine and triethanolamine can be used.

For the water-soluble polymers with basic nitrogen atoms having lone pair electrons at the polymer backbone or side chain, water-soluble polymers selected from polyvinylamine, polyarylamine, polyethyleneimine and polyvinylpyridine.
However, polyethylene glycol, polyvinylalchol, polyacrylamide, polyvinylpyrrolidone and polyvinylacrylic acid did not prevent the formation of silver chloride (AgCl), nor did not prevent the photo-discoloration of the solution. Accordingly, amine or imine compounds with basic nitrogen atoms having lone pair electrons are recommended for the strong coordination with the silver ion.
The water-soluble polymer with basic nitrogen atom having lone pair electrons at the backbone or side chain of the present invention can be prepared by mixing vinylamine, arylamine, ethyleneimine or vinylpyridine in organic solvents like benzene and toluene in the range of 5-20%, and radical-polymerizing them at 60-80°C for 12-24 hrs using BPO as an initiator.
For the silver (Ag) compound coordinating with basic nitrogen, the water-soluble silver compound, especially soluble in ammonia water, is recommended since it easily coordinates with polymers containing amine group. The silver compound with low toxicity is more recommendable for the purpose of the present invention. For example, silver nitrate, silver acetate, silver sulfate, silver benzoate, silver salicylate, silver thiosalicylate or silver sulfadiazine are such silver compounds. A content of silver compounds in the antibacterial agent according to the present invention is in the range of 5-50wt% of the water-soluble polymers with basic nitrogen atoms having lone pair electrons at the polymer backbone or side chain. This content can be adjusted by adjusting the number of lone pair electrons of the basic nitrogen containing amine compounds or polymers.

The present invention includes an antibacterial and deodorizing solution comprising the said silver complex as effective component. Since the antibacterial agent according to the present invention is highly soluble in water, water or mixture of water and alcohol can be used for the solvent, and the solvent can be selected depending on the purpose of use. The preparation method of the antibacterial and deodorizing solution or the effective content of the antibacterial agent is not limited specifically. Also, the common additives such as sodium benzoate, sodium salicylate or colloidal silica can be included: And other additives can be easily used with the common knowledge in the art.

Because the silver complex according to the present invention easily forms complex with offensive-smelling compound or pollutant like dioxin, and prevents its vaporization. So, it helps to prevent offensive odor and environmental pollution. Especially, because silver (Ag) compounds easily forms complex with amine and thiol compounds, they can easily remove amine and thiol offensive-smelling compounds. Accordingly, since the antibacterial and deodorizing solution containing these silver complexes as effective component have superior antibacterial effect, they are useful for the removal of offensive odor caused by microbes like bacteria and molds. Also, they help to prevent the environmental pollution since they prevent the vaporization of offensive-smelling compounds and environmental pollutants by forming complex.

In addition, the silver antibacterial and deodorizing solution of the present invention is useful for the treatment of bums, prevention and treatment of bedsore and treatment of dermatitis because it provides no resistance and very low toxicity. Namely, while the general silver complexes are not suitable for the use as skin-related treatment in spite of their antibacterial effect because they react with the skin protein to discolor the skin black, the antibacterial agent of the present invention is suitable for the use as skin-related treatment in spray, ointment or gel form because it forms complex with amine polymers and the silver is kept in ionic form.

Also, the silver complex antibacterial agent of the present invention can be applied for the fungicide of vegetables and crops or green algae inhibitor of aquarium because they have superior exterminating effect of molds and algae. Generally, microbes proliferate in the stems of flowers with stems, which disturb the supply of nutrients and water through stem and therefore wilt the flowers. The antibacterial agent of the present invention extends the life of flowers by preventing the bacterial infection.

The silver complex antibacterial agent of the present invention is also useful for the extermination of insects like cockroaches. Though its cause is not certain as yet, it may be because the insects have poor digestive function compared with higher animals and can only digest low-molecular weight organic materials. Namely, if the antibacterial agent of the present invention is sprayed on food, the insects may avoid it because they cannot digest it due to the inhibition of microbial growth.

Hereunder is given the more detailed description of the present invention using examples. However, they should not be construed as limiting the scope of the present invention.

### Example 1: Silver-polyethyleneimine complex

After adding 0.5g of silver sulfadiazine, 1g of sodium benzoate and 1g of sodium salicylate in 1L of water, 10g of polyethyleneimine (M.W. 25,000) was added slowly. After stirring sufficiently for 24hr, polyethyleimine complex containing silver salt was obtained.

### Example 2: Silver-polyvinylamine complex

After adding 10g of polyvinylamine and 0.5g of colloidal silica in 1L of water, 2g of silver acetate, 1g of silver thiosalicylate, 10g of sodium benzoate and 1g of sodium salicylate were slowly added while stirring. After stirring sufficiently for 24hr, polyhvinylamine complex containing silver thiosalicylate was obtained.

### Example 3: Silver-triethanolamine complex

After adding 10g of triethanolamine in 1L of water and dissolving it completely, 2g of silver benzoate, 10g of sodium benzoate and 1g of sodium salicylate were slowly added while stirring. After stirring sufficiently for 24hr, triethanolamine complex containing silver benzoate was obtained.

### Example 4: Silver-diethanolamine complex

After adding 10g of diethanolamine in aqueous alcohol solution comprising 800mL of water and 200mL of ethanol, and dissolving it completely, 2g of silver nitrate, 10g of sodium benzoate and 1g of sodium salicylate were slowly added while stirring. After stirring sufficiently for 24hr, diethanolamine complex containing silver salt was obtained.

### Experimental Example 1: Investigation of discoloration resistance

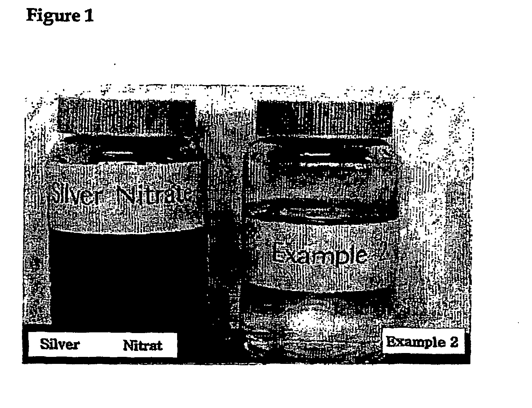

The silver complexes obtained in the above Examples 1-4 were diluted to 2% (containing 10-40ppm of silver ion) with tap water. Silver compounds including silver nitrate, silver sulfide, silver acetate and silver benzoate were dissolved in tap water to 40ppm to be used as contrast groups.
After exposing the solutions to UV for 2hr the discoloration was investigated through eye, and the result is shown in the following Table 1. Also, the photographs after UV exposure of silver nitrate solution and silver-polyethyleneimine complex solution prepared in Example 2 are shown in FIG. 1.

**Table 1**

| Samples | | Discoloration |
|---|---|---|
| Silver Compounds | Silver nitrate solution | 1 |
| | Silver sulfide solution | 1 |
| | Silver acetate solution | 1 |
| | Silver benzoate solution | 1 |
| Silver Complexes | Silver-polyethyleneimine complex (Example 1) | 3 |
| | Silver-polyvinylamine complex (Example 2) | 3 |
| | Silver-triethanolamine complex (Example 3) | 3 |
| | Silver-diethanolamine complex (Example 4) | 3 |
| Note) 1: Black precipitation formed, 2: Thick yellow, 3: Pale yellow, 4: No discoloration | | |

As shown in Table 1 and FIG. 1, the general silver compound solution forms white colloids and then black precipitates afterwards. On the other hand, the silver complex of the present invention shows little photo-discoloration.

### Experimental Example 2: Investigation of minimum growth inhibition concentration

The minimum growth inhibition concentration was measured using test-tube dilution method in order to evaluate the antibacterial activity of the silver complexes obtained in Examples 1-4. The result is shown in the following Table 2.

**Table 2**

| Microbes | Minimum Growth Inhibition Concentration (ppm) | | | |
|---|---|---|---|---|
| | Example 1 | Example 2 | Example 3 | Example 4 |
| *E. coli* (*Escherichia coli* ATCC 25922) | <1 | < 1 | 1 | 1 |
| *E. coli* O-157 (*Escherichia coli* ATCC 43895) | 1 | 1 | 3 | 3 |
| *Staphylococcus aureus* ATCC 25923 | 2 | 3 | 5 | 6 |
| *Staphylococcus aureus* ATCC 6538P | 3 | 3 | 5 | 7 |
| *Trichophyton rubrum* ATCC 28188 | 1 | 2 | 2 | 2 |
| Algae (*Candida Albicans* ATCC 11651) | 1 | 1 | 1 | 1 |
| Yeast (*Trentopholia odorata*) | <1 | <1 | <1 | <1 |

As shown in Table 2, all the silver complexes according to the present invention have superior antibacterial effect.

### Experimental Example 3: Investigation of antibacterial effect on fabrics

After diluting the silver complexes obtained in Examples 1-2 to 2% (containing 10-40ppm of silver ion) with aqueous alcohol solution comprising 9:1 of tap water and ethanol, it was sprayed on cotton fabric. After drying the fabric, its antibacterial effect was investigated. The result is shown in the following Table 3.
The samples used for antibacterial effect evaluation test were 1g respectively, and their size was 10×10×1mm³. The antibacterial effect evaluation test followed the bacteria count method (KS K 0693) using *E*. *coli* ATCC 25922 and *Staphylococcus aureus* ATCC 6538. The bacteria culture time was 24hr.

**Table 3**

| | *E*. *coli* | | | *Staphylococcus aureus* | | |
|---|---|---|---|---|---|---|
| Samples | Initial Number of Microbes | Number of Microbes after Test | Microbial Reduction Ratio (%) | Initial Number of Microbes | Number of Microbes after Test | Microbial Reduction Ratio (%) |
| Example 1 | 1.0×10⁷ | 0 | 100 | 1.9×10⁷ | 2.3×10⁴ | 99.9 |
| Example 2 | 1.0×10⁷ | 9,000 | 99.9 | 1.9×10⁷ | 3.4×10⁴ | 99.8 |

As shown in Table 3, all the silver complexes according to the present invention showed superior antibacterial effect on fabrics.

### Experimental Example 4: Evaluation of fungus resistance on fabrics

After diluting the silver complexes obtained in Examples 1-2 to 2% (containing 10-40ppm of silver ion) with aqueous alcohol solution comprising 9:1 of tap water and ethanol, it was sprayed on cotton fabric. The fungus resistance of the fabric was investigated. The result is shown in Table 5.
The base sample used for fungus resistance evaluation test was 30×30×1mm³. The fungus resistance evaluation test followed the mold resistance test method (KS A 0702) using ATCC 10254 (*Aspergillus niger* ATCC 10254) black mold. The bacteria culture time was 2 weeks.

The mold resistance is presented in the following Table 4.

**Table 4**

| Growth of Hypha | Mold Resistance |
|---|---|
| No hypha growth was identified in the inoculation part of the sample. | 3 |
| The hypha growth area of the inoculation part was smaller than 1/3 of the entire area. | 2 |
| The hypha growth area of the inoculation part was larger than 1/3 of the entire area. | 1 |

The result of the fungus resistance evaluation test is shown in the following Table 5.

**Table 5**

| Samples | Evaluation Result |
|---|---|
| Example 1 | 3 |
| Example 2 | 3 |

As shown in Table 5, the silver complexes according to the present invention have superior mold resistance of 3.

### Experimental Example 6: Evaluation of flower wilting prevention effect

In two vases with the same size 300mL of tap water was poured. 1mL of 2% solution (containing 10ppm of silver ion) of the silver complex obtained from Example 1 was added to one of the two vases and the other was used as a contrast group. After putting 10 as-picked roses in each vase at the same place, the condition of flowers was checked.
The result showed that the flowers in the vase where the antibacterial and deodorizing agent of the present invention was added wilted later than 7 days compared with the contrast group. And, while there was offensive smell in the water of the contrast group vase, the one in the vase wherein the antibacterial and deodorizing agent was added had no offensive odor at all.

### Experimental Example 7: Evaluation of cockroach extermination effect

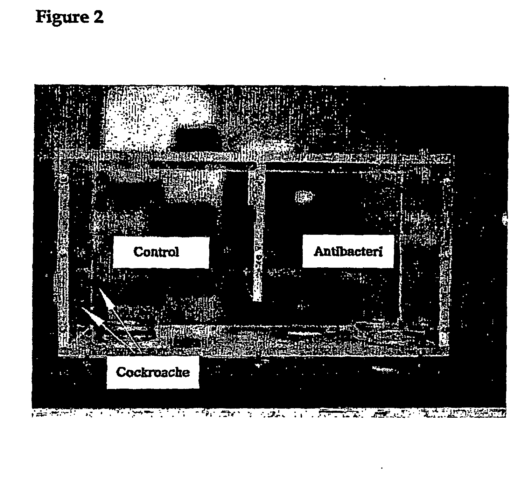

In each side of an acryl box (25×50×25cm³) comparted at the center, bread without sugar was inserted. 2% solution (containing 10ppm of silver ion) of the silver complex obtained from Example 1 was sprayed on one side, and the other was used as a contrast group. After inserting 20 cockroaches in the box, their behavior was observed. The result showed that while all the cockroaches did not stay long at the bread stuck with the antibacterial and deodorizing agent (Example 1) of the present invention, they stayed long and eat the bread in the contrast group. Also, most cockroaches inhabited the chamber where the antibacterial and deodorizing agent was not sprayed. The result is shown in FIG. 2.

### Experimental Example 8: Preparation of ointment

After mixing 1wt% of silver complex obtained from Example 1, 2wt% of wax, 63wt% of pure water and 25wt% of glycerin, they were heated to 65-70°C while stirring for good mixing and dissolving. After adding 10wt% of glyceryl monostearate and heating to 65-70°C, it was stirred with the mixture sufficiently to obtain an ointment.
The fungus resistance was investigated with the same method in Experimental Example 3 to evaluate its efficiency. As a result, it showed very superior antibacterial effect with rating 3.

As explained in detail above, while most silver compounds cannot be used for everyday antibacterial agent in spite of their good antibacterial effect because their color easily changes in common water like tap water or industrial water and discolor the skin; the antibacterial agent of the present invention is a silver complex wherein the lone pair electrons of nitrogen atom in the amine compounds with high boiling point or water-soluble polymers with basic nitrogen are coordinated with silver ion, and it has superior discoloration resistance and antibacterial effect, and highly soluble in water. So, when it is used in liquid form dissolved in water or mixture of water and alcohol, it can be used effectively for removing offensive odor, agricultural fungicide, environmental pollutant elimination, prevention and treatment of bedsore, treatment of dermatitis, antibacterial disinfection, prevention of flower wilting, extermination of cockroaches and prevention of green algae.

## Claims

1. An antibacterial and deodorizing solution comprising a silver complex, wherein the lone pair electrons of nitrogen atom of (i) amine compound with high boiling point selected from monoethanolamine, diethanolamine and triethanolamine or (ii) water-soluble polymer having basic nitrogen atom in the backbone or side chain selected from polyvinylamine, polyarylamine, polyethyleneimine or polyvinylpyridine, are coordinated with silver ion as effective compound.

2. The antibacterial and deodorizing solution according to claim 1, wherein said silver complex is dissolved in water solvent or mixture solvent of water and alcohol.

3. The antibacterial and deodorizing solution according to claim 1, wherein said silver ion is derived from silver nitrate, silver acetate, silver sulfate, silver benzoate, silver salicylate, silver thiosalicylate or silver sulfadiazine.

4. Use of the antibacterial and deodorizing solution according to claims 1 to 3 as deodorizing solution, as antibacterial solution, as antifungal solution, for environmental pollutant elimination, for antibacterial disinfection, for the prevention of flower wilting, for the extermination of cockroaches and for the prevention of green algae.

5. Use of the antibacterial and deodorizing solution according to claims 1 to 3 for the preparation of a medicament for the prevention and treatment of bedsore and for the treatment of dermatitis.

## Patentansprüche

1. Antibakterielle und desodorierende Lösung, die einen Silberkomplex umfaßt, wobei die Elektronen des freien Elektronenpaares des Stickstoffatoms (i) einer Aminverbindung mit hohem Siedepunkt, ausgewählt aus Monoethanolamin, Diethanolamin und Triethanolamin oder (ii) eines wasserlöslichen Polymers mit einem basischen Stickstoffatom im Rückgrat oder der Seitenkette, ausgewählt aus Polyvinylamin, Polyarylamin, Polyethylenimin oder Polyvinylpyridin, mit Silberionen als wirksame Verbindung koordiniert werden.

2. Antibakterielle oder desodorierende Lösung gemäß Anspruch 1, wobei der Silberkomplex in Wasser oder einem Lösungsmittelgemisch aus Wasser und Alkohol aufgelöst ist.

3. Antibakterielle und desodorierende Lösung gemäß Anspruch 1, wobei das Silberion abgeleitet ist aus Silbernitrat, Silberacetat, Silbersulfat, Silberbenzoat, Silbersalicylat, Silberthiosalicylat oder Silbersulfadiazin.

4. Verwendung der antibakteriellen und desodorierenden Lösung gemäß der Ansprüche 1-3 als desodorierende Lösung, als antibakterielle Lösung, als antimycotische Lösung, zur Beseitigung von Umweltverschmutzung, zur antibakteriellen Desinfektion, zur Verhinderung des Welkens von Blumen, zur Ausrottung von Kakerlaken und zur Verhinderung von grünen Algen.

5. Verwendung der antibakteriellen und desodorierenden Lösung gemäß den Ansprüchen 1-3 zur Hierstellung eines Medikaments zur Vorbeugung oder zur Behandlung von Dekubitus und zur Behandlung von Dermatitis.

## Revendications

1. Solution antibactérienne et désodorisante comprenant un complexe d'argent, dans laquelle les électrons d'un doublet libre de l'atome d'azote de (i) un composé amine à haut point d'ébullition choisi parmi la monoéthanolamine, la diéthanolamine et la triéthanolamine ou (ii) un polymère hydrosoluble ayant un atome d'azote basique dans le squelette ou une chaîne latérale choisi parmi la polyvinylamine, la polyarylamine, le polyéthylèneimine ou la polyvinylpyridine, sont coordonnés avec l'ion argent comme composé efficace.

2. Solution antibactérienne et désodorisante selon la revendication 1, dans laquelle ledit complexe d'argent est dissous dans un solvant aqueux ou un solvant mixte d'eau et d'alcool.

3. Solution antibactérienne et désodorisante selon la revendication 1, dans laquelle ledit ion argent est dérivé du nitrate d'argent, de l'acétate d'argent, du sulfate d'argent, du benzoate d'argent, du salicylate d'argent, du thiosalicylate d'argent ou de la sulfadiazine d'argent.

4. Utilisation de la solution antibactérienne et désodorisante selon les revendications 1 à 3 comme solution désodorisante, comme solution antibactérienne, comme solution antifongique, pour l'élimination des polluants environnementaux, pour la désinfection antibactérienne, pour la prévention du flétrissement des fleurs, pour l'extermination des cafards et pour la prévention des algues vertes.

5. Utilisation de la solution antibactérienne et désodorisante selon les revendications 1 à 3 pour la préparation d'un médicament pour la prévention et le traitement des escarres et pour le traitement de la dermatite.
